# EUROPEAN PATENT APPLICATION

(11) **EP 4 413 959 A1**
(43) Date of publication of application: **14.08.2024**
(21) Application number: 23461513.6
(22) Date of filing: 09.02.2023
(51) Int. Cl.: A61F 5/042, A61F 5/37, A61G 13/02, A61B 17/56, A61G 13/12

(54) **DEVICE FOR REDUCTION OF FOREARM FRACTURES AND SET OF DEVICES FOR TREATMENT OF FOREARM FRACTURES**

(71) Applicant: Medirpintic Spolka Z Organiczona Odpowiedzialnoscia, 39-300 Mielec (PL)
(72) Inventor: KOSTUJ, Marcin, 36-100 Kolbuszowa (PL); ZAKRECKI, Andrzej, 31-271 Krakow (PL); PRZYDZIAL, Wojciech, 39-120 Sedziszow Malopolski (PL); CIESLA, Lidia, 36-105 Cmolas (PL)
(74) Representative: AOMB Polska Sp. z.o.o.

(57) **Abstract**

The subject-matter of the invention is a device for reduction of forearm fractures, comprising a base, a body (12) disposed on the base with a palm traction unit (14), a forearm support unit (15), and an arm support unit (16) arranged in series along the axis of the device. The body (12) has a cavity (121) with linear guiding elements (123) arranged essentially along the axis of the device and in a horizontal plane. The palm traction unit (14) and the forearm support unit (15) are slidably arranged on the linear guiding elements (123) of the cavity (121). The palm traction unit (14) is composed of a lower part (149) being a base, a middle part (147), an upper part (145), and a fixing plate (141) comprising finger gripping means, wherein the individual parts are pivotally connected to each other, the middle part (147) is rotatably mounted about the z axis in the lower part (149), wherein the upper part (145) is pivotally connected to the middle part (147) about the y axis, and the fixing plate (141) is pivotally connected to the upper part (145) about the x axis, wherein the palm traction unit (14) is provided with mechanisms for adjusting the angle of rotation of the fixing plate (141), respectively, in the xy plane for the middle part (147), in the xz plane for the upper part (145), in the zy plane for the fixing plate (141. The forearm support unit (15) is composed of a backrest (151) and a housing (152) connected by a lift (153), and a base plate (154) located on linear guiding elements (123) in the cavity (121), wherein the housing (152) contains a drive mechanism of the lift for adjusting the height of the backrest (151). The arm support unit (16) is formed of: a vertical column (161) having on the side facing the cavity (121) at least one vertical guiding element (165); a slider (163) in the form of an elongated element located horizontally and slidably connected to at least one vertical guiding element (165); a biceps holder (164) slidably connected to said slider (163) along its axis.

The subject-matter of the invention relates also to a set of devices for treatment of forearm fractures comprises a device for reduction of forearm fractures and a medical scanner comprising: a base in the form of a stand (23); a movable arm (21) fastened on the stand and mounted rotatably on a horizontal axis of rotation, a counterweight (26) slidably mounted on one end of the movable arm (21); a 3D sensor (22) for performing a 3D scan, placed at the other end of the movable arm (21); and a control device (24) for controlling the 3D sensor (22).

## Description

### Technical field

The present invention relates to the field of medical devices for reduction and treatment of forearm fractures. More particularly, the invention relates to a device for reduction of forearm fractures, referred to also as the reduction device, allowing the fracture reduction process to be accelerated and improved. The invention relates also to a set of devices for treatment of forearm fractures comprising a device for reduction of fractures and a medical scanner.

### Background art

A wide variety of solutions related to medical devices for treatment, including reduction, of forearm fractures are known in the art.

Many types of bone fractures, especially fractures of the upper and lower limb, require immobilization, usually by applying a plaster cast, to ensure proper union. In addition, in the case of displaced fractures, immobilization alone is not sufficient - it is necessary to reduce the fracture before immobilization, so as to obtain the correct position of the bone, as before the fracture.

Compared to other bones of the upper and lower limbs, the bones of the forearm are characterized by a very high mobility in many planes, which makes the reduction of fractures of the forearm bones complicated and time-consuming. When the patient does not have much muscle mass in the forearm area, the surgeon usually adjusts the bone manually without the use of additional equipment. The correct alignment of the displaced parts in this way can be difficult, as even with great care small forearm movements occur that can result in fateful errors. Consequently, after placing the limb in a cast, it may turn out that the reduction was not performed correctly or that it was disturbed during the cast application. On the other hand, in the case of large displacements or when the patient's muscle mass is large, the surgeon uses a traction device for reduction. Most often, the arm is immobilized at the elbow joint and the surgeon pulls the arm to reduce the fracture. Such a system is unstable and dependent on many factors, especially human factors, and therefore large errors can occur during the reduction. Errors may also occur at the stage of cast application, especially since it is applied to the forearm not supported by any stable element.

One of the best-known devices for reduction of forearm (and wrist) fractures is the traction apparatus called the Soko 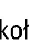 owski apparatus. This apparatus consists of a shaft placed on a stand (or designed to be attached to a hospital bed), to which a crane is telescopically connected, which is clamped at the appropriate height with a jaw lock in order to fix the appropriate extension. The angle of inclination of the shaft can be set with a tilt lock, and its extension with a jaw lock. Attached to the shaft, for example by means of a screw, is a support for the arm, to be placed on it near the elbow joint. The crane is in the form of a rod bent essentially at the right angle. A pulley with a pull cord is attached to the crane. Alternatively, through the hole at its end a traction screw with nut is passed, which in its lower part is connected via a ball joint to a guiding block. An arched channel is made in the guiding block for attaching the guide bar hoop of the traction strap - the traction strap is therefore attached to the traction screw or, in another embodiment, to the end of the pull cord. The fixed and the movable part of the arched clamping jaws are attached to the guide bar. The palm fingers of the hand being set, specifically the proximal phalanges below the joint, are fixed and clamped between these clamping jaws, wherein the position of the hand is adjustable by means of the screw locking the ball joint and the clamping screws of the guide bar hoop. The strength of the traction is adjusted by tightening the nut of the traction.

The clamping jaws are often provided with a flexible material that reduces pressure on the blood vessels, for example, a silicone rubber jacket. However, despite the use of such material, the pressure is still quite strong, so that shortly after the fingers are clamped between the jaws, they turn blue and swell, which causes discomfort and even severe pain. This is particularly noticeable for patients with fractures where a very high traction force must be applied to reduce them. In addition, the use of this apparatus requires the patient to be placed in a supine position and a vertical positioning of the forearm. In individuals suffering from asthma or circulatory system diseases (circulatory failure), this position results in worsening of symptoms of the diseases. In addition, in the Soko 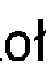 owski apparatus, the pulling force acts in the axis of the forearm being reduced, which results in straightening of the hand in the wrist joint, which in turn is an undesirable position for reduction of many types of fractures, for example, Colles fractures. Therefore, the reduction of some types of fractures with the use of this apparatus is not possible, and the only available remaining option is surgical reduction. Due to its construction, such a device also makes it difficult to apply an orthopaedic dressing (plaster or orthosis) directly on the fracture and limits the reduction of fractures of the wrist and forearm bones, due to the lack of an additional element stabilizing the forearm and the inability to adjust pronation and supination.

Another device for reduction of forearm bones, operating in a vertical plane, is disclosed in US7771378B2. It is a reduction device in the form of a tower, which has a base for receiving the patient's arm on it, with means of fixation for immobilizing the elbow joint, a vertically positioned arm and a joint that movably connects the base and the arm. The arm at its upper end has traps for four fingers (except the thumb), made in the form of cover tips. At the height of the wrist, the arm has an adjuster allowing the angle of inclination of the arm to be changed so that it is possible to adjust the position of the palm relative to the forearm. However, such a device still requires the forearm to be aligned in a vertical position, and the finger traps used may cause excessive stretching of the fingers and pain.

Considering the described drawbacks associated with the use of devices operating in a vertical plane, it is preferred that a device for reduction of forearm fractures operates in a horizontal plane. The operation in a horizontal plane enables good control of the reduction process and allows the size of the device to be reduced.

An example of a device for forearm fracture reduction operating in a horizontal plane is a device disclosed in Polish patent PL167602B1, consisting of a shaft having two parallel arms. An arm stabilizing strap is attached to one of the arms, and to the other - a traction finger trap and a holder fixing the device to the operating table. The shaft is made of two parts, each of them consisting of an L-shaped pair of tubes permanently connected to each other. The tubes are slidably connected with their longer ends arranged horizontally, while their shorter ends set upwards form the vertical arms of the shaft. A forearm support unit is mounted on the horizontal part of the shaft. The shaft is movably vertically and rotatably connected to the setting unit, and further to the holder fixing the device to the table. An arm stabilizing strap is attached to the vertical arm of the shaft, rotatably about the axis of the forearm. The traction finger trap and the traction thumb trap are attached through the traction arch to the second vertical arm, rotatably about the axis of the forearm and movably vertically and horizontally.

On the other hand, CN103750888B discloses a reduction device produced in the form of a frame, comprising, inter alia, a hand fixing frame and an elbow support. The device is provided with an examination hoop which is perpendicular to the horizontal plane that passes through the frame of the device. An examination opening is facing the radius and the wrist of the patient and allows for checking if the reduction is correct and for imageological examination of the injury without the need to release the limb from the device.

It is also known in the art to use X-rays to scan fractures before or during the reduction of the limb, which makes it easier for the doctor to recognize the fracture and, as a result, allows him to adjust the activities performed during the reduction. Devices are also known to scan a limb as an instruction to an automatic reduction device.

And so, the Chinese patent document CN106491196B discloses a radius reduction and force measurement device, operating in a horizontal plane. The device comprises a forearm and elbow joint supporting system, a biplanar arc-shaped guide rail system, an upper arm fixing system, a thumb traction system and a palm traction system. The guide rail system and the upper arm fixing system are arranged on the forearm and elbow joint supporting system, and the thumb traction and the palm traction systems are arranged on the biplanar guide rail system. The reduction device can cooperate with an X ray emitting device, so that a doctor can monitor the effects of his/her work on an ongoing basis.

On the other hand, CN103750888B discloses a method and system for automatic limb fracture reduction. The method comprises a step of scanning the limb with X rays. The fracture information is processed by computer so that a limb fracture model and a movement trajectory required by the fracture reduction are obtained, and the trajectory is reproduced by an automatic traction device conducting the traction reduction of the fractured limb.

Methods for fabricating orthoses based on a 3D model created with the use of a medical scanner are also known in the art.

WO2021156894A1 discloses a method of fabricating a personalised orthopaedic cast that includes 3D scanning of a body part of a user, generating a computer aided design (CAD) of an orthopaedic cast for the scanned body part, and simulating real-life conditions to determine mechanical stability.

EP3359036B1 discloses a method to generate recommendations for orthoses and includes receiving information from a user (identification of limbs). On-screen cues are provided to assist in positioning of given extremity areas relative to sensors. A model of a given extremity area is generated based on the data from the scanning process. This solution therefore includes the selection of the appropriate type of orthosis from a certain database.

EP3662872A1 discloses a system for manufacturing an orthosis to be fitted on a human body on the basis of 3D data. The system includes the storage of the manufacturing history data relating to other orthoses manufactured in the past, and an orthosis formation unit on the basis of the 3D data.

Summarizing the known state of the art, solutions are known that combine the use of a reduction device with a medical scanner, wherein the scanner is used to X-ray the fracture in order to obtain its image, and as a result, to facilitate reduction - by a doctor or a reduction device. Solutions are also known for selecting an orthosis model from a certain database on the basis of fracture input data, as well as devices that create an orthosis model based on a 3D scan of the limb.

However, none of the known devices allows for a sufficiently good control of the reduction process, especially due to the lack of correct stabilization of the forearm that ensures adequate rigidity of holding the hand, and also due to the lack of necessary setting of the wrist inclination angle, specific for some types of fractures. None of the known solutions allows for simultaneous fracture reduction and creation of a virtual model or selection of an appropriate orthosis.

In addition, at present, during the therapeutic procedure in the case of a forearm fracture, after applying a plaster cast, the patient is sent for a follow-up X-ray. If everything went well, the patient is discharged home, and if the X-ray image shows that the bone fragments have moved, the orthopaedist may decide on a renewed reduction attempt after removing earlier the plaster cast, or refer the patient to surgery. The reason for failures may be an imprecise reduction of the fracture, which leads to displacements during plastering or in the process of plaster drying. It also happens that the fragments move already after reduction. This consumes valuable time of employees involved in the treatment and involves large financial resources due to the need for further treatment and convalescence of the patient. In addition, applying a plaster cast is burdensome and inconvenient for the doctor and the patient - due to a vertical plane of action of many known reduction devices, the plaster flows along the patient's limb.

### Summary of the invention

The subject-matter of the present invention relates to a device for reduction of forearm fractures, comprising a base, a body disposed on the base with a palm traction unit, a forearm support unit, and an arm support unit arranged in series along the axis of the device, wherein
the body has a cavity with linear guiding elements arranged essentially along the axis of the device and in a horizontal plane;
the palm traction unit and the forearm support unit are slidably arranged on the linear guiding elements of the cavity;
the palm traction unit is composed of a lower part being a base, a middle part, an upper part, and a fixing plate comprising finger gripping means, wherein the individual parts are pivotally connected to each other, the middle part is rotatably mounted about the z axis in the lower part, wherein the upper part is pivotally connected to the middle part about the y axis, and the fixing plate is pivotally connected to the upper part about the x axis, wherein the palm traction unit is provided with mechanisms for adjusting the angle of rotation of the fixing plate, respectively, in the xy plane for the middle part, in the xz plane for the upper part, in the zy plane for the fixing plate;
the forearm support unit is composed of a backrest and a housing connected by a lift, and a base plate located on linear guiding elements in the cavity, wherein the housing contains a drive mechanism of the lift for adjusting the height of the backrest;
the arm support unit is formed of: a vertical column having on the side facing the cavity at least one vertical guiding element; a slider in the form of an elongated element located horizontally and slidably connected to at least one vertical guiding element; a biceps holder slidably connected to said slider along its axis.

In one embodiment, the palm traction drive comprises a column with a drive wheel disposed on the body, a bevel gear disposed inside the column and a worm gear disposed in the body and coupled to the palm traction unit.

In one embodiment, the fixing plate is provided with the palm finger gripping means in the form of five longitudinal grooves that extend radially and five grips each slidably arranged in the longitudinal groove.

In one embodiment, the forearm support unit is provided with a brake for blocking the movement along the linear guiding elements in the cavity.

In another embodiment, the arm support unit includes a slider height adjustment mechanism comprising an adjustment wheel with a handle disposed on top of the column and mounted on the upper end of the power screw, and a helical gear whose nut is connected to the slider.

In yet another embodiment, the biceps holder is connected by a slide bearing to the slider carriage, which slider carriage is slidably mounted on the guide rail of the slider.

In yet another embodiment, the base of the body is provided with a lifting mechanism for adjusting the distance of the body from the ground, the range being preferably from 510 mm to 910 mm.

The subject-matter of the present invention relates also to a set of devices for treatment of forearm fractures comprising a device for reduction of forearm fractures and a medical scanner, said scanner comprises:
a base in the form of a stand;
a movable arm fastened on the stand and mounted rotatably on a horizontal axis of rotation,
a counterweight slidably mounted on one end of the movable arm;
a 3D sensor for performing a 3D scan, placed at the other end of the movable arm;
and a control device for controlling the 3D sensor.

In one embodiment, the arm is composed of a first part, a second part and a third part, wherein the first part with the second part and the second part with the third part are movably connected via a first joint and a second joint, respectively, and wherein the 3D sensor is movably connected to the third part.

### Advantageous effects of the invention

The reduction device according to the invention allows the forearm fracture reduction process to be accelerated and improved in a manner controlled by a doctor. The device operates in a horizontal plane, which allows for a good control of the reduction process. The supports for stabilizing the forearm in three points of support, which are part of the reduction device, ensure that the hand is held rigid and enable the reduction of such types of fractures that cannot be reduced using devices known in the art. The reduction device can be integrated with a medical scanner that is used to scan externally the injured forearm, and then to create a virtual model of the orthosis on this basis. The integration of the reduction device with the scanner ensures that a precise virtual model is obtained by ensuring the image stability - when using handheld scanners, the patient is not able to keep the broken forearm in one position for longer than 3 minutes. The integration of the scanner with the reduction device allows also the forearm to be scanned in the correct position of the palm relative to the forearm, which results from the reduction of the fracture just before the scan is performed. The scanning time is no longer than 45 seconds, which is much less compared to the commercially available solutions.

### Brief description of the drawings

Fig. 1 shows the device for reduction of forearm fractures (reduction device);
Fig. 2 shows the trolley of the reduction device;
Fig. 3 shows a view of the main drive;
Fig. 4 shows the main drive in top view;
Fig. 5 shows a fragment of the reduction device with the palm traction unit and the main drive;
Fig. 6 shows a view of the palm traction unit;
Fig. 7 shows the palm traction unit in side view, from the side facing the long side of the body, i.e. perpendicular to the x axis from Fig. 6;
Fig. 8 shows the palm traction unit in side view, from the side facing the forearm mechanism, i.e. perpendicular to the y axis from Fig. 6;
Fig. 9 shows the palm traction unit in top angle view;
Fig. 10 shows the forearm support unit in side view, from the side facing the long side of the body;
Fig. 11 shows a view of the forearm support unit, placed in the body;
Fig. 12 shows a view of the arm support unit;
Fig. 13 shows the arm support unit in top view;
Fig. 14 shows the arm support unit in side view, from the side facing the long side of the body;
Fig. 15 shows the arm support unit in side angle view, with visible elements of the connection of the biceps grip with the slider;
Fig. 16 shows the arm support unit in side view, from the side equipped with vertical rails;
Fig. 17 shows the medical scanner;
Fig. 18a,b shows a fragment of the scanner in the place where its arm is connected to the post of the stand, before and after connecting said elements, respectively;
Fig. 19 shows a fragment of the scanner including the counterweight and the parts to which it is attached.

### Detailed description

The set being the subject of the present invention consists of a device for reduction of forearm fractures, referred to as a reduction device, and a medical scanner. The reduction device can also be used alone, i.e. without a medical scanner.

The reduction device allows for aligning the upper limb in the right position, moving bone parts and/or bone fragments into the right places. The reduction is an activity that restores the correct alignment and position of bone fragments, including the reconstruction of dented cancellous bone fragments and fragments of articular surfaces. The reduction reverses the processes that displaced the fragments during the injury and requires the use of opposing forces. Regardless of whether the fracture is simple, comminuted, multilevel or with bone defects, the goal of reduction is to restore the correct length, axis and rotation of the bone and joint system, so that the bones and adjacent joints are aligned as they were before the fracture.

The reduction device is equipped with a support for stabilizing the forearm in three points of support, ensuring that the hand is held rigid and enabling the reduction of various types of fractures. Applying a dressing or an orthosis using this device is possible in a stable position of the forearm, including immobilization of the arm and hand fingers - the reduction device stabilizes the patient's hand in a position in which the dressing should be applied. When operating the device, the doctor sets the upper limb in the reduction device, and then, through the adjustment mechanisms, aligns the hand in the right position, moving the bone fragments into the right places.

As already mentioned earlier, the reduction device can be integrated with a medical scanner, with which it creates a set of devices for the treatment of forearm fractures, the function of which is to scan the forearm or wrist (collecting biometric data), and then to create a virtual model that serves as a basis for fabrication of an orthosis model. The reduction device additionally acts as a hand stabilizer for the time of scanning. The virtual model is developed on the basis of a cloud of points collected during the scanning, then the scanner software converts the cloud of points into an STL model. On the basis of the obtained virtual STL model, the orthosis model is produced. It is possible to use the scanner to indicate to the doctor the appropriate orthosis, adapted to the type of injury suffered. The integration of the reduction device with the scanner ensures that a precise virtual model is obtained by ensuring the image stability and allows the forearm to be scanned in the correct position of the hand in relation to the forearm. The scanning time is no longer than 45 seconds, which is much less compared to the commercially available solutions. The device can be controlled by using, for example, a tablet or laptop.

A more detailed description of an embodiment of the reduction device, the scanner and both as a set will be provided below with reference to the accompanying Figures.

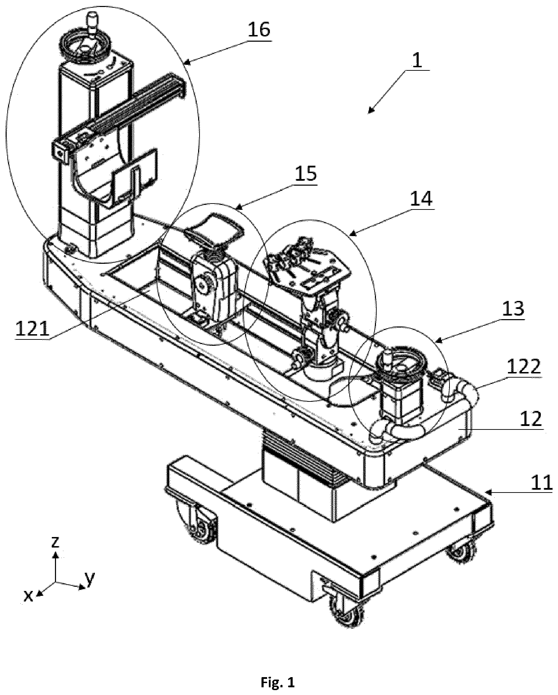

A view of a practical embodiment of the reduction device 1 is shown in Fig. 1. The reduction device 1 is essentially formed of the following elements: a base in the form of a trolley 11, a body 12 of a generally cuboidal shape with a cavity 121 in its upper part and connected to the body 12, and arranged in series along the axis of the body 12: a palm traction drive 13, a palm traction unit 14, a forearm support unit 15, and an arm support unit 16. The palm traction drive 13 and the arm support unit 16 are attached to the two extreme ends of the body 12. Located between them are the palm traction unit 14 and the forearm support unit 15, wherein the palm traction unit 14 is located next to the palm traction drive 13, and the forearm support unit 15 is located next to the arm support unit 16. The body 12 is also optionally equipped with a handle 122 for gripping and displacement.

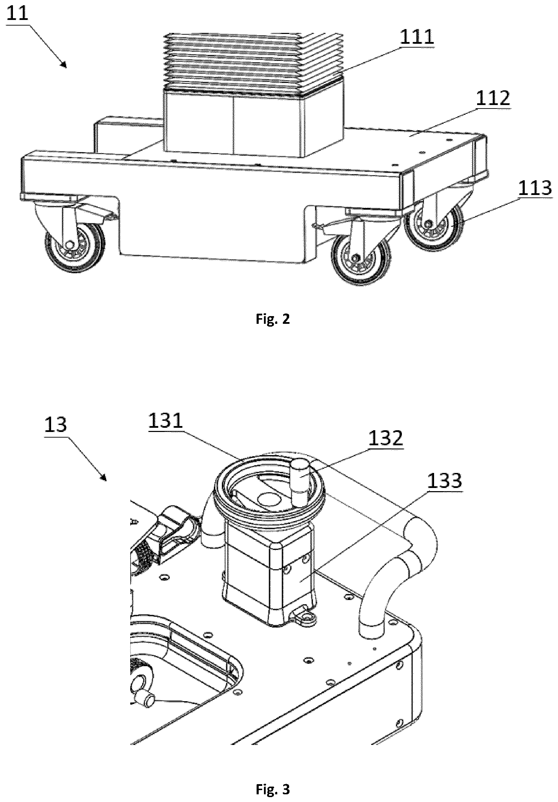

Fig. 2 shows the base of the body 12 in the form of a trolley 11. The trolley 11 is disposed in the lower part of the reduction device 1 and is attached to the underside of the body 12. The trolley 11 is equipped with a lifting mechanism 111 for adjusting the position of the body 12, i.e. to adjust the distance of the body 12 from the ground. The lifting mechanism 111 may be pneumatic or mechanical and may, for example, be implemented as a lift, as can be seen in Fig. 2. The height adjustment range of the body 12, defined as the distance of this body 12 from the ground, in the embodiment is from 510 mm to 910 mm. The trolley 11 is provided with a plate 112 to which said lifting mechanism 111 is firmly attached. Also fixed to the plate 112 are means for moving the trolley 11, for example wheels 113. In another embodiment, the base of the body can be provided with a lifting mechanism 111, wherein it does not need to be made in the form of a trolley 11 - in other words, it can be a stationary base equipped with a lifting mechanism 111. In yet another embodiment, the base can be devoid of a lifting mechanism 111 - in this case, the adjustment of the height of the patient's position relative to the reduction device 1 is carried out by adjusting the height of the couch (or the treatment table, treatment bed, hospital bed, etc.) on which the patient lies.

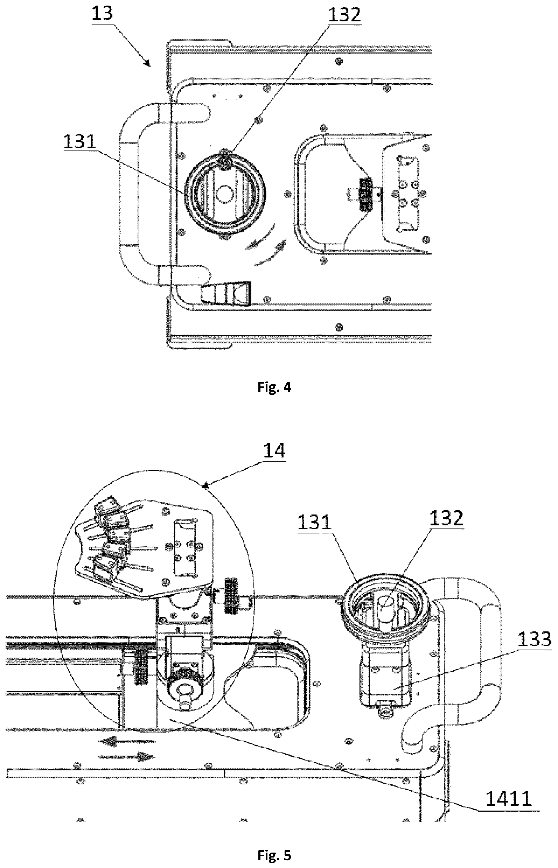

To the body 12, in the extreme position (at one of its shorter ends), a palm traction drive 13 is attached, as shown in Fig. 3 and in a top view in Fig. 4. The palm traction drive 13 is used to apply a traction to the upper limb. The palm traction drive 13 in the embodiment is comprised of a drive wheel 131 provided with a handle 132 for rotating the drive wheel 131 around its axis, and a column 133 on top of which the drive wheel 131 is disposed. The palm traction drive 13 is connected to the palm traction unit 14 through a bevel gear located in the column 133 and a helical gear located in the body 12, the feed screw nut of which is attached to the sliding plate 1411, on which the elements of the palm traction unit 14 are mounted. The traction is carried out by the linear movement of the palm traction unit 14, forced by rotations of the drive wheel 131. The relative alignment of the palm traction drive 13 and the palm fixation mechanism 14 is shown in Fig. 5. Also shown in Fig. 5 are the directions of movement of the palm traction unit 14 during rotation of the drive wheel 131 in both directions, wherein the rotation of the drive wheel 131 is also marked with arrows in Fig. 4. The range of travel of the palm traction unit 14 along the axis of the reduction device (as indicated by the arrows in Fig. 5), i.e. relative to the y axis of the Cartesian coordinate system, is 600 mm.

In another embodiment, the column 133 of the palm traction drive 13 with the drive wheel 131 may be attached to either side of the body 12. In yet another embodiment, the linear movement of the palm traction unit 14 may be manually operated (in which case the reduction device 1 will be devoid of the palm traction drive 13). The linear movement of the palm traction unit 14 may also be effected in any other known manner, such as by means of an electric motor operated by push buttons. In such cases, the range of travel of the palm traction unit 14 is also, for example, 600 mm.

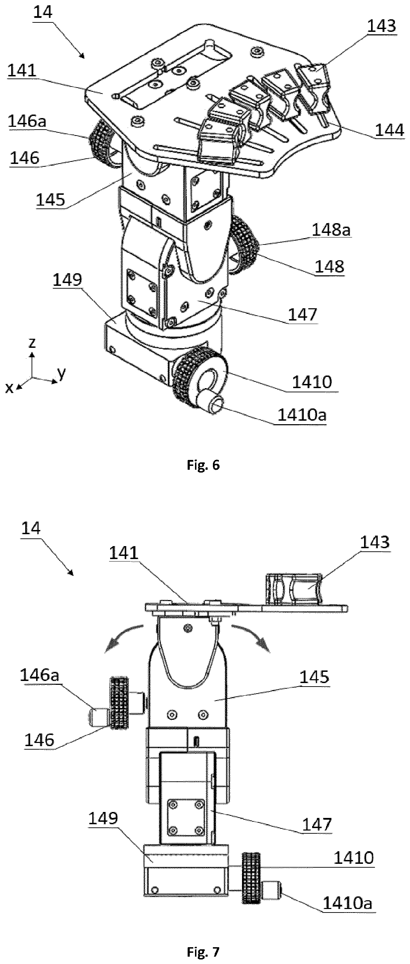

The palm traction unit 14 is shown from different perspectives in Fig. 6 to Fig. 9. The function of this unit is to fix the patient's palm and to enable its re-positioning, i.e. to change the angle of inclination of the palm at the wrist, by controlling the angles of rotation relative to the three axes of the Cartesian coordinate system (xyz ), as marked, for example, in Fig. 1 and 6.

The palm traction unit 14 it is equipped with a fixing plate 141 and a shaft 142 divided into three parts: an upper part 145, a middle part 147, and a lower part 149. The fixing plate 141 is used to fix the palm stably, and for this purpose it is equipped with five finger traps 143, each of which is located in a longitudinal groove 144. The finger traps 143 are slidably located in the longitudinal grooves 144, i.e. they can be moved along these grooves 144 to fit the fastening to the patient's palm. The longitudinal grooves 144 extend radially over a sector of a circle. The fixing plate 141 is attached to the upper part 145 of the shaft 142 and mounted pivotally thereon, making a rotation about the x axis.

The adjustment of the angle of inclination of the fixing plate 141 is carried out by means of a fixing plate knob 146 with a knob handle 146a disposed in the upper part 145 and coupled by a worm gear to the fixing plate 141. The fixing plate knob 146 is used to adjust the angle of inclination of the fixing plate 141 around the x axis, i.e. in the yz plane as shown in Fig. 7 (side view). The adjustment range of the rotation angle of the fixing plate 141 for the vertical position is +90° / -90°.

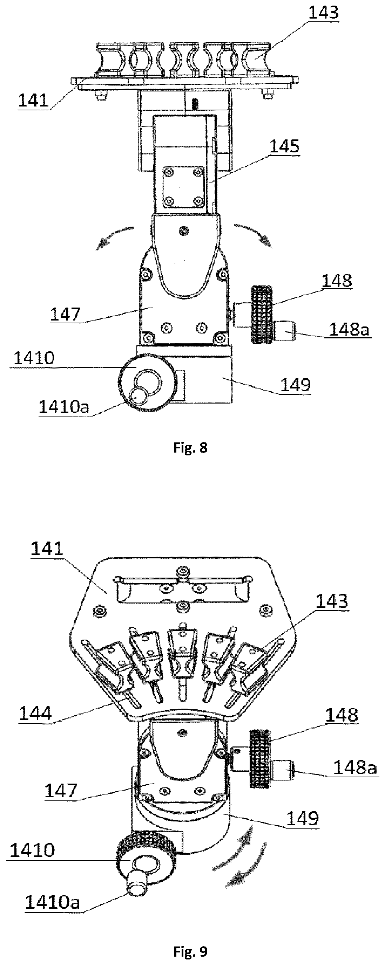

The upper part 145 of the shaft 142 is pivotally connected to the middle part 147. The upper part 145 rotates about the y axis, i.e. in the xz plane. The adjustment of the inclination of the upper part 145, and thus of the fixing plate 141 connected thereto, is carried out by means of a upper part knob 148 with a knob handle 148a disposed in the middle part 147 and coupled by a worm gear with the upper part 145. The upper part knob 148 is used to adjust the angle the inclination of the upper part 145, and thus indirectly the angle of inclination of the fixing plate 141, about the y axis, i.e. in the xz plane, as shown in Fig. 8 (side view). The adjustment range of the rotation angle of the upper part 145 for the vertical position is +90° / -90°.

The middle part 147 of the shaft 142 is connected to its lower part 149. The angle of rotation of the middle part 147 is adjusted by means of a middle part knob 1410 with a knob handle 1410a disposed on the side of the lower part 149 and coupled with the middle part 147 as shown in Fig. 9 ( angled top view).

In an embodiment, the lower part 149 is fixed on the sliding plate 1411 (Fig. 5). The middle part 147 is rotatably connected to the fixed lower part 149, wherein the adjustment range of the rotation angle of the middle part 147 around the z axis (in the xy plane) is +45° / -45°.

The mutual movement of the elements described above, i.e. the upper part 145, the middle part 147, and the lower part 149, is carried out by worm gears operated by the aforementioned knobs 146, 148 and 1410, wherein these gears are self-locking, so that the individual parts, after being set in a given position, lock automatically.

The height of the palm fastening on the fixing plate 141, i.e. the distance of the fixing plate 141 from the body 12, for the initial ("zero") position is 270 mm. The initial position is one in which all parts of the palm traction unit 14 are aligned vertically in one line.

On both sides of the sliding plate 1411, linear carriages are attached thereto and move along linear guiding elements 123, for example suitably profiled guide rails, which are described in more detail below.

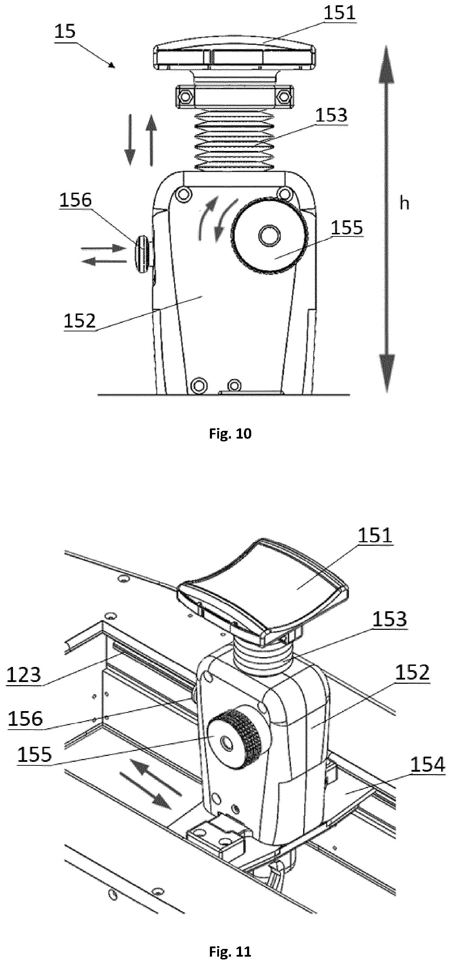

The forearm support unit 15 is shown in Fig. 10 (in side view) and in Fig. 11 (disposed in the cavity 121 of the body 12). The function of the forearm support unit 15 is to stabilize and position the forearm appropriately, depending on the type of fracture and the patient's body structure. The forearm support unit 15 consists of a backrest 151 on which the forearm (or the elbow joint) is placed, a housing 152, a lift 153 arranged between the backrest 151 and the housing 152, and a sliding plate 154 on which the housing 122 is disposed. In the housing 152 a drive mechanism of the lift 153 is mounted, which allows the backrest 151 to be raised and lowered, thus adjusting its height. The drive mechanism of the lift 153 is preferably made as a rack-and-pinion drive including a toothed bar and a toothed wheel that engages an adjustment knob 155 disposed on the side of the housing 152. The lift 153 is preferably provided with a ratchet mechanism (not shown) in the form of a pin 156 terminated with a ratchet, which engages with an additional ratchet wheel mounted on an axle that connects the toothed wheel of the drive mechanism to the adjustment knob 155. The pin 156 protrudes from the side of the housing 152. Insertion of the pin 156 into the housing 152 locks the position of the lift 152 in a given position, and retraction thereof from the housing results in releasing the ratchet mechanism, which allows the backrest 151 to be lowered downwards. The height adjustment range of the backrest 151 relative to the body 12, marked "h" in Fig. 10, is from 225 mm to 355 mm.

As is clearly seen in Fig. 11, the sliding plate 154 of the forearm support unit 15 is coupled in the cavity 121 of the body 12 with the linear guiding elements 123, which allows it to be moved along the axis of the device. For this purpose, the sliding plate 154 may be provided with guided elements, for example linear carriages, which slide over these linear guiding elements 123. The range of movement of the forearm support unit 15 along the axis of the reduction device 1, indicated by arrows in Fig. 11, is 550 mm. The movement of the forearm support unit 15 in the cavity 121 of the body 12 is preferably carried out manually and the locking is performed by means of a brake. If required (depending on the type of fracture), a substantial part of the forearm support unit 15 can be removed by unscrewing the screws connecting the slide plate 154 to the housing 152.

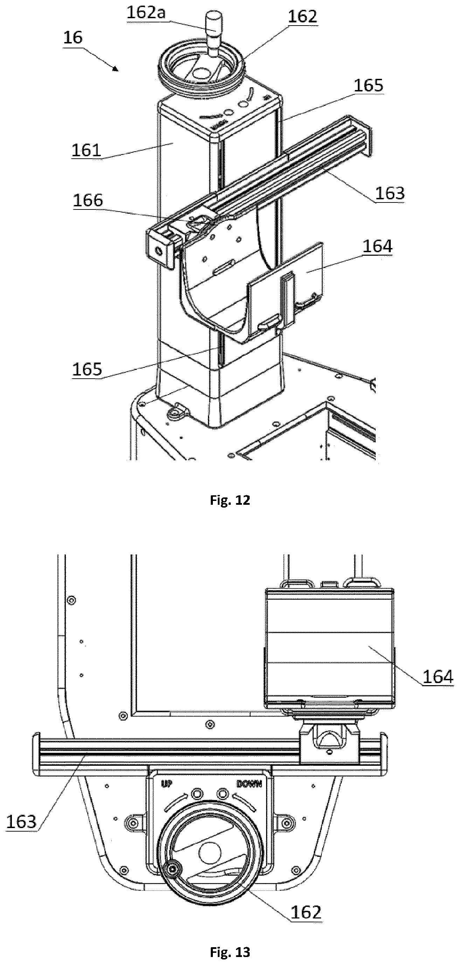

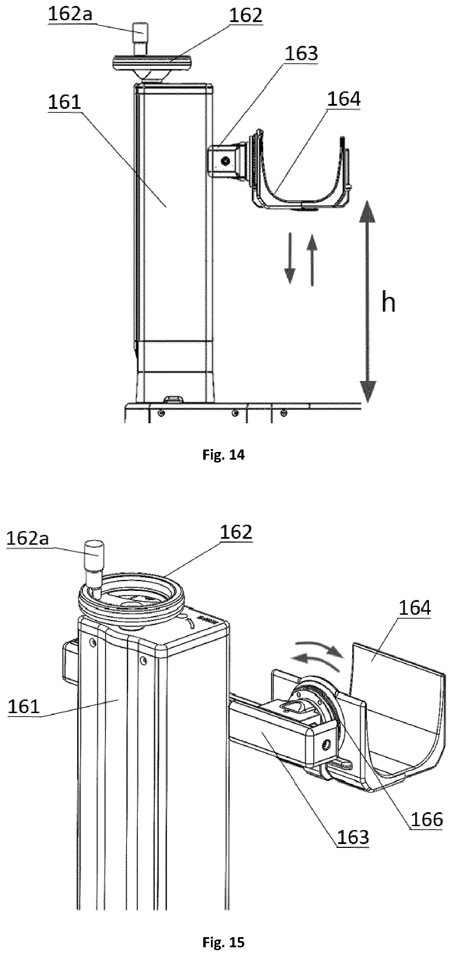

Figs. 12 to 16 show the arm support unit 16 in different views. The function of the arm support unit 16 is to control the position of the biceps (the height, the distance from the axis of the device and the angle of rotation). The arm support unit 16, shown in perspective view in Fig. 12, includes a vertical column 161, on top of which there is an adjustment wheel 162 with a handle 162a. The arm support unit 16 further comprises a slider 163 to which a biceps holder 164 is attached. On the side of the column 161 facing the cavity 121 of the body 12, linear guiding elements are formed, more specifically two vertical guide rails 165. The slider 163 is connected by complementary guided elements to said vertical guide rails 165. The height of the position of the slider 163 and the biceps holder 164 is adjusted by means of a drive mechanism in the form of a helical gear, the feed screw nut of which is coupled with the slider 163. In an embodiment, turning the adjusting wheel 162 to the right results in rotation of the feed screw and movement of the feed screw nut and thus the slider 163 upwards, while turning the adjusting wheel 162 to the left moves the slider downwards. The height adjustment range of the position of the biceps holder 164 relative to the body 12 of the reduction device 1, marked "h" in Fig. 14, is from 70 mm to 380 mm, wherein this range is given from the body 12 to the base of the biceps holder 164 (i.e. its lower part on which the hand rests). In another embodiment, the adjustment of the height position of the slider 163 in the vertical rails 165 may be carried out manually. The movement of the slider 163 may also be effected in any other known manner, e.g. by means of an electric motor. In these other embodiments, the preferred height adjustment range of the slider position remains the same as mentioned above.

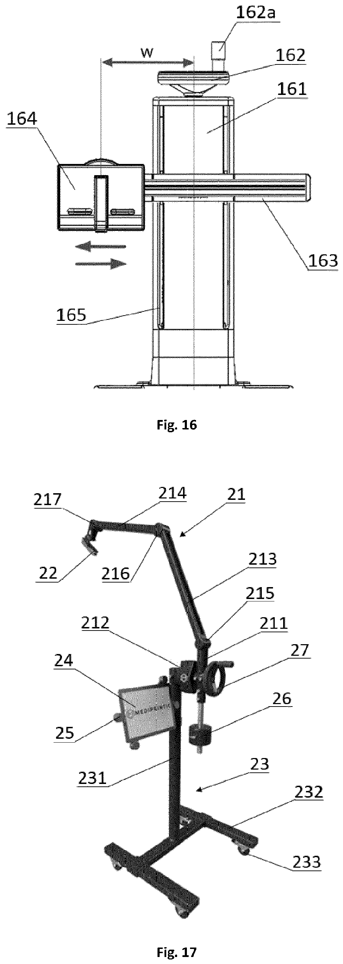

The biceps holder 164 is slidably connected to the slider 163 and moves along the axis of the slider 163. The movement of the biceps holder 164 along the slider 163 is effected manually - it is moved by gripping the biceps holder 164 with hand and sliding it along the rails of the slider 163 (wherein blocking in a given position is carried out e.g. by a brake or a mechanical lock). In addition, the biceps holder 164 is attached to the slider 163 via a slide bearing 166, which is also a swing bearing. This allows the biceps holder 164 to rotate around a horizontal axis and tilt slightly. The connection of the biceps holder 164 to the slider 163 through the slide bearing 166 is clearly visible in Fig. 15. On the other hand, the range of movement of the biceps holder 164 along the slider 163 and from the axis of the column 161 is marked in Fig. 16 "w" and is +/- 165 mm. In Fig. 16, the biceps holder 164 is in a position fully extended to one side of the slider 163.

As already mentioned, the movement of the palm traction unit 14 and the forearm support unit 15 along the axis of the device and in the horizontal plane is carried out by means of guides. For this purpose, linear guiding elements 123 are provided on both opposite vertical walls of the cavity 121, along its long edges. The linear guiding elements 123 may be basically of any form that provides linear movement. They can therefore take the form of profiled guide rails, rollers or other shaped surfaces. The linear guiding elements 123 cooperate with the complementary guided elements of the palm traction unit 14 and the forearm support unit. The guided elements may, for example, be linear carriages or slides slidingly connected to the linear guiding elements. Another example of guides that can be used to implement linear motion are rolling guides.

A medical scanner 2, shown in Fig. 17, can be integrated with the reduction device 1 - the reduction device 1 and the scanner 2 then form a set of co-operating devices for treatment of forearm fractures. The function of the medical scanner 2 is to scan the injured forearm placed in the reduction device 1 (thus also acting as a stabilizer in this case), and then to create a virtual model of the forearm on the basis of which an orthosis model for the patient is fabricated. It is therefore possible to scan the limb stabilized in the reduction device 1 immediately - in one place. The medical scanner 2 is thus used to collect biometric data for designing a forearm orthosis. It is also possible to use the medical scanner 2 with its software as a tool for indicating the appropriate orthosis for a given patient, matched to the type of injury suffered (wherein the indication is based on the model of the forearm developed with use of this scanner 2). In addition, it is also possible to combine the reduction device 1 with the mobile C-arm of a portable X-ray device, which allows the doctor to conduct X-ray diagnostics directly in the reduction device 1, and thus correct the alignment of bone fragments in real time in such a way that they are set in the best possible position for the bone union. In other words, the reduction device 1 can be combined with the scanner 2 to create a 3D model of the forearm to select the appropriate orthosis for it, and/or it can be combined with the mobile C-arm to take an X-ray to check whether the forearm reduction process has been completed correctly.

The virtual model of the forearm is created on the basis of a cloud of points collected during scanning. Then the software of the medical scanner 2 (and specifically the 3D sensor) converts the cloud of points into an STL model and performs positioning in the Cartesian system. On the basis of the obtained virtual STL model, a model of the orthosis is fabricated. Alternatively, the STL model of the forearm is compared with the available base of orthoses by percentage adjustment of the position of the forearm to the shape of the orthosis, and on this basis the orthosis appropriate for a given patient is selected - the scanner 2 compares the dimensions of the forearm with the parameters of available orthoses (manufactured by the Applicant), and thus allows for selection of the optimal solution.

The integration of the reduction device 1 with the scanner 2 ensures that a precise virtual model is obtained by ensuring the image stability and allows the forearm to be scanned in the correct position of the palm relative to the forearm, immediately after reduction of the fracture. The scanning lasts for no longer than 45 seconds, and the scanning accuracy (error range) is up to 1 mm.

The medical scanner 2 is shown in full in Fig. 17. The medical scanner 2 consists of a movable arm 21, a 3D sensor 22 (in the embodiment it is a Structure Sensor Pro 3D sensor) disposed at the end of the arm 21, a base in the form of a stand 23 (preferably mobile), a control device 24 (in the embodiment it is a tablet, iPad Air A2316) placed in the holder 25, a connection cable (e.g. Sensor Pro <--> USB type C), an extension cable, (the connection cable and the extension cable are not shown in the drawing), a counterweight 26 and an arm position adjuster 27. The stand 23 is composed of a vertical post 231 and a mobile part 232, preferably in the form of a trolley equipped with wheels 233. The stand 23 can also be composed of a vertical post 231 disposed on a stationary base. In the embodiment, the movable arm 21 is formed of three parts: a first part 211 provided with a clamp 212, a second part 213 and a third part 214, wherein the first part 211 with the second part 213, and the second part 213 with the third part 214 are movably connected, preferably via a first joint 215 and a second joint 216, respectively. A 3D sensor 22 is movably connected to the third part 214, for example by means of a third joint 217.

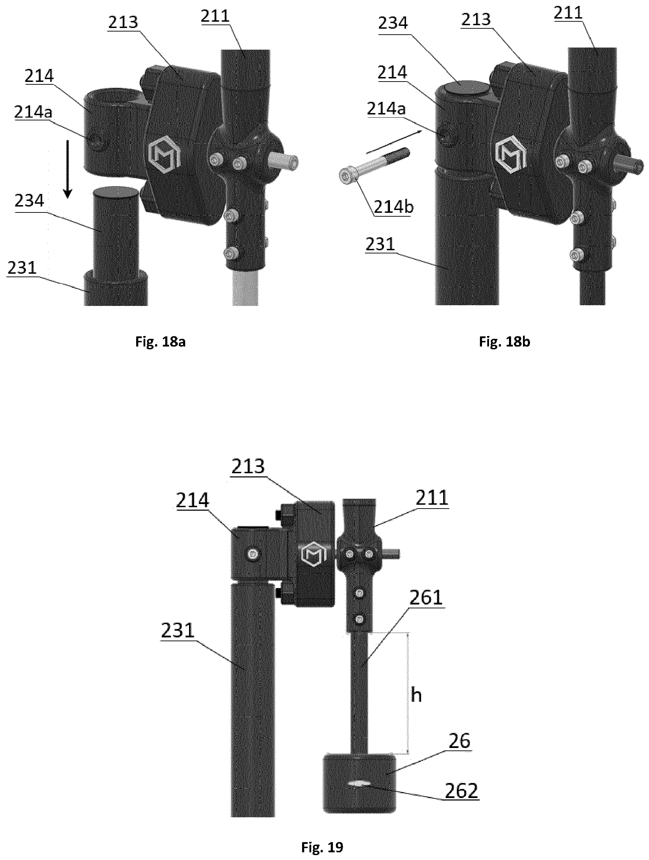

The first part 211 of the arm 21 is used to connect the arm 21 to the post 231 of the stand 23, as shown in Fig. 18. For this purpose, the post 23 ends with a tapered end in the form of a pin 234, and the first part 211 is provided with a connecting element 212 comprising a connector 213 and a sleeve-shaped clamp 214 (see Fig. 18a). The clamp 214 is connected to the first part 211 through a connector 213, the connector 213 and the clamp 214 forming one element, connected, for example, by a screw connection to the first part 211. In another embodiment, said elements may be monolithic. The diameter of the clamp 214 matches the diameter of the tapered end 234 so that the tapered end 234 can be inserted into the clamp 214. The clamp 214 is provided on both opposite sides with a hole 214a for a mounting screw 214b. Also, the tapered end 234 has a hole 234a for the mounting screw 214b that aligns with the hole 214a of the clamp 214. When the clamp 214 is applied to the tapered end 234, the clamp 214 rests on the thicker part of the post 231, and the mounting screw 214b is inserted into the holes 214a, 234a (Fig. 18b). Preferably, the mounting screw 214b is an M8 screw, which is locked with a nut when screwed in. In another embodiment, the connection of the stand 23 to the arm 21 may be implemented reversely, i.e. the first part 211 of the arm 21 may be provided with a tapered end, and the post 231 may end with a clamp. When connecting the clamp 214 to the post 231, especially when tightening the mounting screw 214b, particular care must be taken because of the fragility of the joined elements, which are preferably fabricated by 3D printing.

The arm 21 is movable and for this purpose it is provided with means for adjusting its position. In an embodiment, the arm 21 is provided with an adjuster to control its rotation to the left and to the right.

In another embodiment, the medical scanner may not be equipped with a stand 23. In this case, the arm 21 is provided with connecting means for connecting the arm to the reduction device 1 or to the table (couch) on which the patient lies.

The movable arm 21, and specifically in the embodiment its first part 211, has at its lower end a counterweight 26, which is clearly visible in Fig. 19. The counterweight 26 is located at a suitable distance from the first part 211 ("h" in Fig. 19) and its position is adjustable. In the embodiment, it is a (vertical) distance from 20 mm to 120 mm from the end of the arm 21 (here: the first part 211), depending on the needs. The counterweight 26 is slidably mounted on the axis 261 and secured with a set screw 262. The axis 261 is attached with its other end to the first part 211, and is preferably fastened thereto with screws. In order to balance the movable arm 21 during scanning, the counterweight 26 should be placed 20 mm from the first part 211. After scanning, it is necessary to lower the counterweight 26 to a distance 120 mm from the first part 211 in order to prevent the arm 21 from losing its balance and falling down.

The connection cable is connected on one side to the 3D sensor 22 and on the other side to the control device 24. The connection between the 3D sensor is provided, for example, with a USB type C cable. The connection cable may be routed in a tray grooved in the third part 214 and the second part 213 of the arm 21. The 3D sensor 22 is separately powered by a charger. An extension cable is connected to the control device 24 to charge it.

In order to facilitate the proper positioning of the medical scanner 2, the joints 215, 216, and 217 of the arm 21 are each indicated with a suitable position, for example by markers, the alignment of which in one line corresponds to the correct positioning of the parts of the arm 21 relative to each other.

The reduction device 1 and the medical scanner 2 are made of materials suitable for this purpose, i.e. stainless steel, structural steel, aluminium, rubber-like materials, plastics such as ABS or PA12 polyamide, PA-CF. The plastic parts are preferably produced by 3D printing.

An application ("Mediprintic"), operated by the control device 24, preferably a tablet, placed in the holder 25 attached to the stand 23 (specifically, to the post 231), is dedicated to control the medical scanner 2. The control device 24 may also be a laptop or smartphone, as well as a desktop computer (the control device 24 need not be placed, of course, in the handle 25, if its size makes this inconvenient).

The application operated by the control device 24 is designed to control the operation of the 3D sensor 22, process the obtained scan, create a model of the forearm and create/select an orthosis based on this model.

The course of the procedure for operating the reduction device 1 or the set of the reduction device 1 and the scanner 2, i.e. performing the fracture reduction and scanning the limb, is as follows.

The patient is placed on his back, with his shoulder girdle completely outside the couch on which the patient lies. The next step is to prepare the reduction device for placing the hand in it. Then sedation is started and anaesthesia is applied according to the applicable procedures.

The next step is repositioning. The direction of the applied forces, achieved by properly positioning the components of the reduction device, depends on the type of fracture. The distal bone fragment should be moved to the proximal fragment. The forearm rotation should be corrected to match the proximal fragments. A correct patient positioning is a necessary condition for achieving an adequate traction. Accurate positioning of the shoulder joint in abduction of 90° and flexion of the elbow joint to 90° allow for traction in the axis passing through the fracture site. The next step is to reduce the fracture.

Optionally, after fracture reduction, in the next step it is possible to perform a follow-up radiological examination using a mobile C-arm (portable X-ray machine). The mobile C-arm is a standard equipment in the treatment room where the process of forearm reduction is carried out. Anteroposterior (AP) and lateral radiographs of the forearm, wrist, and elbow may be taken to assess the progress of repositioning. If the reduction of fracture was not performed correctly, a correction is carried out. This solution eliminates the step of taking an X-ray in a separate unit - an X-ray room, which translates into cost savings of the procedure and reduction of the number of personnel involved in the course of trauma treatment. Further, the real-time X-ray control of the reduction allows for reduction of the number of repeated fracture reductions, i.e. allows for reduction of the number of procedures performed in relation to incorrect reduction of fractures of the upper limbs and the number of surgical procedures performed due to poor stabilization of bone fragments in the fixation process and application of orthopaedic dressing.

If a scanner 2 is connected to the reduction device 1, the next step is to perform a medical 3D scan using a 3D sensor 22 placed on the arm 21 of the scanner 2, operated by the control device (preferably from the application, the operation of which is described above).

Based on the performed 3D scan, an appropriate orthosis is selected using the software (from the application level). Alternatively, a new orthosis model is created based on the 3D scan, from which subsequently a new orthosis is created. The orthosis is fabricated by 3D printing using known methods. These two functions can also occur simultaneously, i.e. the software can be simultaneously configured to select the orthosis and to create the orthosis model, depending on the needs.

The finished orthosis is then applied to the forearm, wherein the application of the orthosis is carried out while maintaining the traction, which ensures stabilization of the hand and forearm during this step. In other words, if the orthosis is planned to be put on, after fracture reduction, the traction achieved by the reduction device 1 is not released (the hand is still in the reduction device 1 in the position in which it was immediately after the fracture reduction). In another embodiment, it is possible to omit the scanning; then a plaster cast is applied to the forearm, also while maintaining the forearm traction achieved in the reduction device 1, which ensures stabilization for the time of cast application.

The last step of the procedure is a follow-up radiological examination in the AP (anterior-posterior) and lateral projection of the elbow joint, forearm and wrist.

The reduction device described herein as well as the set of the reduction device and the scanner is intended, inter alia, for:
- reduction of intra-articular fractures and simple fractures of the wrist and forearm bones: displaced fractures of the distal radius, fractures of the shaft of the ulna with or without dislocation of the radial head; fractures of the shaft of the radius with or without dislocation of the distal radioulnar joint, stable and non-displaced fractures of the scaphoid and distal radius; fractures of both forearm bones, fractures of the shaft of the fourth or fifth metacarpals, fractures of the neck of the fourth or fifth metacarpals);
- reduction of dislocations and subluxations within the wrist: sprains and dislocations of the radiocarpal joint, mid-carpal joint and metacarpocarpal joints; lesser arch injuries - perilunar or lunate dislocation, greater arch injuries - perilunar or lunate dislocation with concomitant wrist fractures;
- intraoperative treatment of displaced fractures of the wrist and forearm bones according to the Müller AO classification using the open method;
- and for intraoperative use in surgical procedures within the wrist and forearm bones (e.g. wrist arthroscopy).

The main contraindication to manual reduction of displaced bone fragments are inveterate fractures, multiple fractures or fractures accompanying injuries to multiple organs (polytrauma).

## Claims

1. A device for reduction of forearm fractures, comprising a base, a body (12) disposed on the base with a palm traction unit (14), a forearm support unit (15), and an arm support unit (16) arranged in series along the axis of the device, **characterized in that**
the body (12) has a cavity (121) with linear guiding elements (123) arranged essentially along the axis of the device and in a horizontal plane;
the palm traction unit (14) and the forearm support unit (15) are slidably arranged on the linear guiding elements (123) of the cavity (121);
the palm traction unit (14) is composed of a lower part (149) being a base, a middle part (147), an upper part (145), and a fixing plate (141) comprising finger gripping means, wherein the individual parts are pivotally connected to each other, the middle part (147) is rotatably mounted about the z axis in the lower part (149), wherein the upper part (145) is pivotally connected to the middle part (147) about the y axis, and the fixing plate (141) is pivotally connected to the upper part (145) about the x axis, wherein the palm traction unit (14) is provided with mechanisms for adjusting the angle of rotation of the fixing plate (141), respectively, in the xy plane for the middle part (147), in the xz plane for the upper part (145), in the zy plane for the fixing plate (141);
the forearm support unit (15) is composed of a backrest (151) and a housing (152) connected by a lift (153), and a base plate (154) located on linear guiding elements (123) in the cavity (121), wherein the housing (152) contains a drive mechanism of the lift for adjusting the height of the backrest (151);
the arm support unit (16) is formed of: a vertical column (161) having on the side facing the cavity (121) at least one vertical guiding element (165); a slider (163) in the form of an elongated element located horizontally and slidably connected to at least one vertical guiding element (165); a biceps holder (164) slidably connected to said slider (163) along its axis.

2. The device for reduction of forearm fractures according to claim 1, **wherein** the palm traction drive (13) comprises a column (133) with a drive wheel (131) disposed on the body (12), a bevel gear disposed inside the column (133) and a worm gear disposed in the body (12) and coupled to the palm traction unit (14).

3. The device for reduction of forearm fractures according to either claim 1 or 2, **wherein** the fixing plate (141) is provided with the palm finger gripping means in the form of five longitudinal grooves (144) that extend radially and five grips (143) each slidably arranged in the longitudinal groove (144).

4. The device for reduction of forearm fractures according to any preceding claim, **wherein** the forearm support unit (15) is provided with a brake for blocking the movement along the linear guiding elements (123) in the cavity (121).

5. The device for reduction of forearm fractures according to any preceding claim, **wherein** the arm support unit (16) includes a slider height adjustment mechanism comprising an adjustment wheel (162) with a handle (162a) disposed on top of the column (161) and mounted on the upper end of the power screw, and a helical gear whose nut is connected to the slider (163).

6. The device for reduction of forearm fractures according to any preceding claim, **wherein** the biceps holder (164) is connected by a slide bearing (166) to the slider carriage (167), which slider carriage (167) is slidably mounted on the guide rail (163a) of the slider (163).

7. The device for reduction of forearm fractures according to any preceding claim, **wherein the** base of the body (12) is provided with a lifting mechanism (111) for adjusting the distance of the body (12) from the ground, the range being preferably from 510 mm to 910 mm.

8. A set of devices for treatment of forearm fractures, **characterized in that** it comprises a device for reduction of forearm fractures according to any claim 1 to 7 and a medical scanner (2) comprising:
a base in the form of a stand (23);
a movable arm (21) fastened on the stand and mounted rotatably on a horizontal axis of rotation,
a counterweight (26) slidably mounted on one end of the movable arm (21);
a 3D sensor (22) for performing a 3D scan, placed at the other end of the movable arm (21);
and a control device (24) for controlling the 3D sensor (22).

9. The set of devices for treatment of forearm fractures according to claim 8, **wherein** the arm (21) is composed of a first part (211), a second part (213) and a third part (214), wherein the first part (211) with the second part (213) and the second part (213) with the third part (214) are movably connected via a first joint (215) and a second joint (216), respectively, and wherein the 3D sensor (22) is movably connected to the third part (214).
